(19) Europäisches Patentamt / European Patent Office / Office européen des brevets

(11) **EP 2 782 033 B1**

(12) **EUROPEAN PATENT SPECIFICATION**

(45) Date of publication and mention
of the grant of the patent:
**05.05.2021 Bulletin 2021/18**

(51) Int Cl.:
*G16C 20/30* [(2019.01)]    *G16B 15/30* [(2019.01)]
*G16C 20/50* [(2019.01)]    *G16C 20/64* [(2019.01)]

(21) Application number: **14152546.9**

(22) Date of filing: **24.01.2014**

(54) **Calculation method of binding free energy, calculation device of binding free energy, program, screening method of compound**

Berechnungsverfahren zur Bindung freier Energie, Berechnungsvorrichtung zur Bindung freier Energie, Programm, Screening-Verfahren zur Verbindung

Procédé et dispositif de calcul d'énergie libre de liaison, programme et procédé de criblage d'un composé

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **22.03.2013 JP 2013060097**

(43) Date of publication of application:
**24.09.2014 Bulletin 2014/39**

(73) Proprietor: **FUJITSU LIMITED
Kawasaki-shi,
Kanagawa 211-8588 (JP)**

(72) Inventor: **TANIDA, Yoshiaki
Kawasaki-shi, Kanagawa 211-8588 (JP)**

(74) Representative: **Haseltine Lake Kempner LLP
138 Cheapside
London EC2V 6BJ (GB)**

(56) References cited:
**EP-A2- 2 509 015    WO-A1-2011/016743
US-A1- 2011 130 968**

• PEI J. ET AL: "Estimating protein-ligand binding free energy: Atomic solvation parameters for partition coefficient and solvation free energy calculation", PROTEINS: STRUCTURE, FUNCTION, AND BIOINFORMATICS, vol. 57, no. 4, 18 August 2004 (2004-08-18), pages 651-664, XP055125533, ISSN: 0887-3585, DOI: 10.1002/prot.20198

• ZOU X. ET AL: "Inclusion of Solvation in Ligand Binding Free Energy Calculations Using the Generalized-Born Model", JOURNAL OF THE AMERICAN CHEMICAL SOCIETY, vol. 121, no. 35, 21 August 1999 (1999-08-21), pages 8033-8043, XP055125532, ISSN: 0002-7863, DOI: 10.1021/ja984102p

• MASSOVA I. ET AL: "Combined molecular mechanical and continuumsolvent approach (MM-PBSA/GBSA) to predict ligandbinding", PERSPECTIVES IN DRUG DISCOVERY AND DESIGN, vol. 18, no. 1, June 2000 (2000-06), pages 113-135, XP055125534,

**Description**

The embodiments discussed herein relate to a calculation method and calculation device of binding free energy between a protein and a compound, a program for carrying out the calculation method, and a screening method of a compound using the calculation method.

Simulations using various calculators have currently been carried out in order to reduce enormous cost and efforts required for experimentally searching drug candidate molecules. The search of drug candidate molecules is to search compounds (ligands), which are strongly interacted with a protein associated with a target disease (disease to be targeted), as drug candidates. To this end, screening of compounds based on a special configuration of a protein has been actively performed by means of a calculator.

In the screening, the most stable conformation of a compound, especially the most stable conformation in the state where a compound is interacted with a protein, is evaluated with an energy function, to predict a binding conformation or binding ability. The method for predicting the most stable conformation of a compound varies depending on an approximation level of a calculation, and examples thereof include a molecular orbital (MO) method, a molecular mechanics (MM) method, a molecular dynamics (MD) method, and docking simulation. These methods search a conformation at which the energy becomes the minimum, and binding conformation or binding ability between a protein and a compound (ligand) is predicted based on the searched most stable conformation.

As for a general technique in the screening, disclosed are conformation analysis, high speed docking studies, calculation of binding free energy, and formation of a binding model of a compound with a target protein (see, for example, International Patent Application No. WO2003/038672).

It is very difficult to directly calculate binding free energy between a protein and a drug candidate molecule in a solvent. Therefore, binding free energy is calculated via a virtual state in which a drug candidate molecule is disappeared. In this case, the calculation has been conventionally performed with restricting a protein and a drug candidate molecule with a spring. However, the restriction with the spring involves a calculation step for optimizing a spring constant, and the calculation accuracy thereof is low, such as about $\pm$ 2 kcal/mol. Therefore, there is a problem that it is difficult to predict an experimental value with chemical accuracy (up to 1.4 kcal/mol). Especially, the larger a drug candidate molecule is, the lower the accuracy is.

Accordingly, there is currently a need for a method and device for accurately calculating binding free energy between a protein and a compound, a program for carrying out the calculation method, and a method for efficiently screening a compound using the calculation method.

PEI et al. discloses a computer-implemented method for estimating protein-ligand binding free energy including solvation energy calculation based on atomic solvation parameter (ASP) set. The ASP set is used to develop an algorithm for solvation energy calculation, which is then integrated into a score function for predicting the binding free energy of a protein-ligand complex.

Zou et al. discloses computer-based ligand binding free energy calculations using the generalized-Born model of solvation. This method also accounts for the hydrophobic effect in terms of the change in solvent-accessible surface area during binding. The model is implemented in the DOCK software package providing an additional scoring function, so-called free energy score.

Massova et al. teaches a computational method combining molecular mechanical approaches for the sampling and the continuum electrostatic methods for the solvation free energies. The conformational sampling is carried out with molecular dynamics software. The binding free energy is determined from the free energies of the complex, protein and peptide.

EP 2 509 015 A2 is directed to a computer-implemented method for estimating a free energy difference between interacting compounds in a solvent.

US 2011/130968 A1 discloses a method for computational simulation of ligand-host binding where changes in solvation that accompany ligand binding are taken into account and structures of host molecules are modified to improve estimates of binding energies.

WO 2011/016743 A1 discloses a computer-implemented method for calculating solvation energy using Fast Surface Based Electrostatics (FSBE) approach. It also discloses a method for predicting the solvation energy contribution to the prediction of binding free energy of therapeutic molecules or ligands with biological molecules (e.g., proteins) in aqueous environment.

The present invention aims to solve the aforementioned various problems in the art. Theinvention aims to provide a method for accurately calculating binding free energy between a protein and a compound, and a calculation device, a program for carrying out the calculation method, and a method for efficiently screening a compound using the calculation method.

The disclosed calculation method of binding free energy between a compound and a protein in a solvent comprises, using a computer,:

calculating solvation energy ($\Delta G_1$) between the solvent and the compound; and
calculating an energy change ($\Delta G_2$) between a bound state ($\lambda=0$) where the compound and the protein are bound, and an unbound state ($\lambda=1$) where the compound and the protein are not bound,
wherein the binding free energy is the sum of the solvation energy ($\Delta G_1$) between the solvent and the compound and the energy change ($\Delta G_2$) between the bound state ($\lambda=0$), and the unbound state ($\lambda=1$), and
wherein the calculating the energy change ($\Delta G_2$) includes:

determining a distance ($D_{th}$), within which structure sampling is performed, based on a distance between the compound and the protein in the bound state ($\lambda=0$), wherein the distance ($D_{th}$) is selected from distances between 90% and 100% of a minimum value in a frequency distribution of a distance that is a result obtained by simulating the distance between the compound and the protein in the bound state ($\lambda=0$);
calculating a change in binding energy ($\Delta G_{21}$) between the compound and the protein within a distance equal to or shorter than the distance ($D_{th}$) on a state ($\lambda=\lambda m$) that is a state between the bound state ($\lambda=0$) and the unbound state ($\lambda=1$), the state ($\lambda=\lambda m$) including a state in which a distance between the compound and the protein is equal to or shorter than the distance ($D_{th}$),
calculating a change in solvation energy ($\Delta G_{23}$) between the solvent and the compound in the unbound state ($\lambda=1$) and a state ($\lambda=\lambda n$) that can be regarded as the same as the unbound state ($\lambda=1$), the state ($\lambda=\lambda n$) that can be regarded as the same as the unbound state ($\lambda=1$) being a state between the bound state ($\lambda=0$) and the unbound state ($\lambda=1$) wherein there is hardly any interaction between the compound and the protein,
calculating a change in binding energy ($\Delta G_{22}$) between the compound and the protein on a state between the state ($\lambda=\lambda m$) and the state ($\lambda=\lambda n$), with interpolating from the state ($\lambda=\lambda m$) and the state ($\lambda=\lambda n$), and
calculating a correction term ($\Delta G_{24}$) with respect to a standard state of the compound, based on a volume of a space calculated from the distance ($D_{th}$), the volume being a spherical volume having a radius of the distance ($D_{th}$), the correction term ($\Delta G_{24}$) being calculated according to the formula

$$\Delta G_{24} = -k_B T \left( \frac{V^{site}}{V^\circ} \right)$$

where $V^0$ represents a standard state volume, $V^{site}$ represents a volume of a bind pocket of the protein, T is temperature and $k_B$ is the Boltzmann constant,
wherein the energy change ($\Delta G_2$) is the sum of: the change in binding energy ($\Delta G_{21}$) between the compound and the protein within a distance equal to or shorter than the distance ($D_{th}$) on a state ($\lambda=\lambda m$), the change in binding energy ($\Delta G_{22}$) between the compound and the protein on a state between the state ($\lambda=\lambda m$) and the state ($\lambda=\lambda n$), the change in solvation energy ($\Delta G_{23}$) between the solvent and the compound in the unbound state ($\lambda=1$) and a state ($\lambda=\lambda n$), and the correction term ($\Delta G_{24}$) with respect to the standard state of the compound.

The disclosed screening method of a compound comprises:

calculating binding free energies of a protein with each of a plurality of compounds in accordance with the disclosed calculation method of binding free energy; and
selecting the compound based on the calculated binding free energies, wherein the selected compound is a compound having an optimal value, from among the calculated binding free energies of the plurality of compounds, as a drug candidate molecule suitable for the protein.

Also disclosed is a program for causing a computer to execute calculation of the binding free energy between a compound and a protein in a solvent.
Also disclosed is a device for calculation of binding free energy. The scope of the invention is defined by the claims.
The invention will now be further described, by way of example only, with reference to the following figures, in which:

FIG. 1 is a model diagram of a thermodynamic cycle associated with the energy of ligand molecules A and B and a target protein.
FIG. 2 is a model diagram of a thermodynamic cycle associated with the energy in the state where there is no ligand molecule A in FIG. 1.
FIG. 3 is a model diagram of a thermodynamic cycle associated with the energy in the case where restriction with a spring is used.
FIG. 4 is a graph depicting one example of a result of molecular dynamics calculation related to a distance between a compound and a protein in a bound state (λ=0).

FIG. 5 is a graph depicting one example of a result of molecular dynamics calculation related to a distance between a compound and a protein in a state (λ=λm) that is between the bound state (λ=0) and the unbound state (λ=1).

FIG. 6 is a graph depicting one example of a change in binding energy associated with LJ interaction between a compound and a protein.

FIG. 7 is a diagram illustrating one example of a change in a state of a compound and a protein.

FIG. 8 is a graph depicting one example of a change in binding energy associated with Coulomb interaction between a compound and a protein.

FIG. 9 is a flow chart illustrating one example of the disclosed calculation method of binding free energy.

FIG. 10 illustrates a structural example of a hardware of the disclosed calculation device of binding free energy.

FIG. 11 is a table depicting Example results.

The scope of the invention is defined by the claims.

(Calculation Method of Binding Free Energy)

The disclosed calculation method of binding free energy contains at least a first step and a second step, and may further contain other steps according to the necessity.

The calculation method is a calculation method of the binding free energy between a compound and protein in a solvent.

First, outlines of the disclosed calculation method of binding free energy is explained.

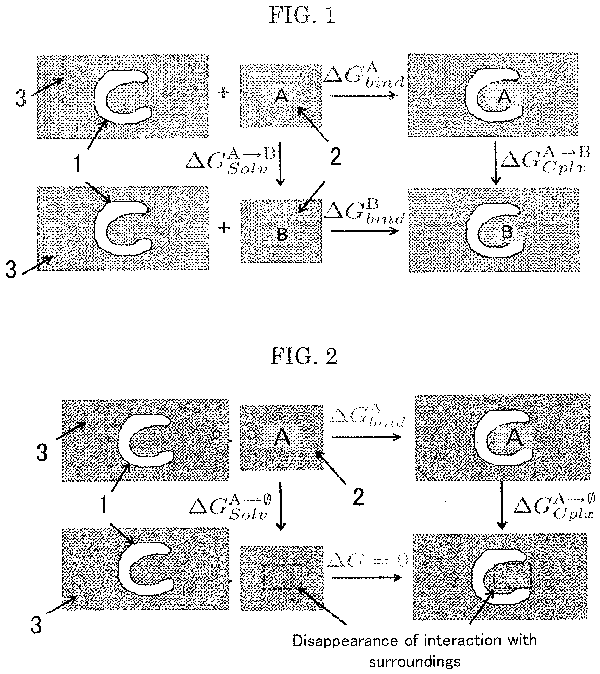

The binding free energy ($\Delta G^A_{bind}$, $\Delta G^B_{bind}$) of a target protein (1) with drug candidate molecule (ligand molecule) A or B (2) can be represented as in FIG. 1. In FIG. 1, the reference number "3" represents water.

The relation between $\Delta G^A_{bind}$ and $\Delta G^B_{bind}$ can be represented as follows in accordance with a thermodynamic cycle.

$$\Delta\Delta G_{bind} = \Delta G^B_{bind} - \Delta G^A_{bind}$$
$$= \Delta G^{A\to B}_{Cplx} - \Delta G^{A\to B}_{Solv}$$

By replacing the state depicted in FIG. 1 with a state where there is no ligand molecule B, with reference to the relation depicted in FIG. 1, binding free energy of a target protein (1) and a drug candidate molecule (ligand molecule A) (2) can be represented as in FIG. 2.

$\Delta G^A_{bind}$ can be represented as follows:

$$\Delta G^A_{bind} = \Delta G^{A\to\emptyset}_{Solv} - \Delta G^{A\to\emptyset}_{Cplx}$$

Here, the energy term ($\Delta G^{A\to 0}_{Solv}$) of the right hand side of the expression above can be calculated by the first step, and the energy term ($\Delta G^{A\to 0}_{Cplx}$) of the right hand side can be calculated by the second step.

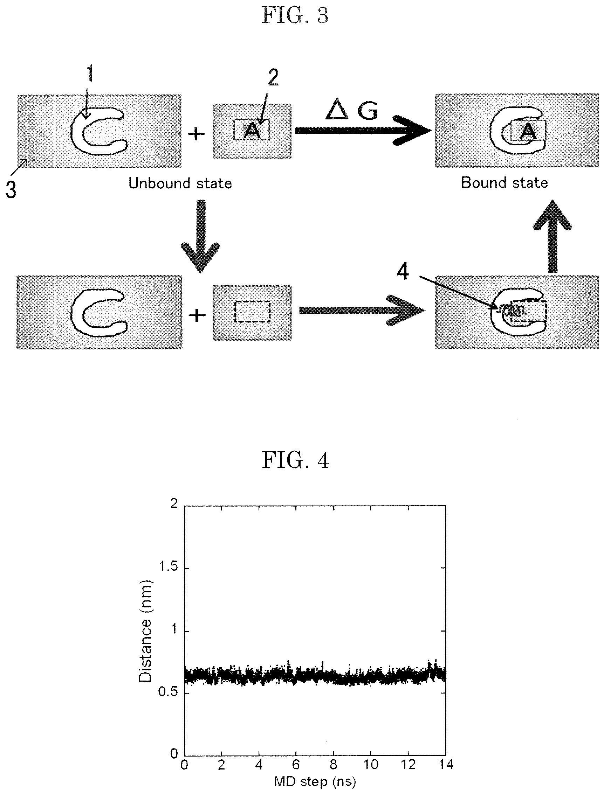

Conventionally, the energy term ($\Delta G^{A\to 0}_{Cplx}$) has been calculated with a model using restriction with a spring (4) (FIG. 3). However, the restriction with a spring (4) involves a calculation step for optimizing a spring constant, and the calculation accuracy is low, such as ± 2 kcal/mol.

In the disclosed calculation method of binding free energy, the restriction with a spring is not performed. Therefore, an accuracy of calculation can be improved.

When the restriction with a spring is simply not performed, a space in which drug candidate molecules are present is increased by a space in which the restriction with a spring occupies otherwise, therefore the space within which structure sampling is performed becomes large.

In order to perform structure sampling with a region where an interaction between a drug candidate molecule and a protein is strong, therefore, an appropriate distance, within which structure sampling is performed, is determined based on a distance between the drug candidate molecule and the protein in the state where the interaction thereof is the strongest (bound state). In the state where the distance between the drug candidate molecule and the protein is within the aforementioned distance, a change in binding energy between the drug candidate molecule and the protein is calculated.

In the region where there is hardly any interaction between the drug candidate molecule and the protein, moreover, the solvation energy of the drug candidate molecule in the solvent is calculated with ignoring an influence of the protein.

Moreover, the region between the region where the interaction is strong and the region where there is hardly any interaction is a region where a change is drastic, and therefore it is difficult to calculate. For this reason, the energy change in such region is calculated by interpolating from the energy change in the region where the interaction is strong and the region where there is hardly any interaction.

Furthermore, a volume correction is performed to compare with an experiment.

In the manner as described above, binding free energy can be accurately calculated.

<First Step>

The first step is appropriately selected depending on the intended purpose without any limitation, provided that the first step is calculating solvation energy ($\Delta G_1$) between the solvent and the compound.

One example of the first step is explained. (1) An interaction between the compound, and the solvent surrounding the compound is determined as a binding constant $\lambda^1$.

Here, a state where the compound is present in the solvent and the compound is completely interacted with the solvent is determined as $\lambda^1=0$, and a state where the compound is present in the solvent but is not interacted with the solvent at all is determined as $\lambda^1=1$.

(2) In order to calculate $\Delta G_1$, the state is divided as $\lambda^1=\{0, \lambda_1, \lambda_2, \lambda_3, \ldots \lambda_{n-1}, 1\}$.

(3) A change in $\Delta G_1$ ($\Delta G_{i \to i+1}$) when the state is changed from $\lambda^1_i$ to $\lambda^1_{i+1}$ is calculated.

(4) $\Delta G_1$ is obtained by adding up all $\Delta G_{i \to i+1}$.

A calculation program used here is appropriately selected depending on the intended purpose without any limitation, and examples thereof include a molecular dynamics calculation program. Examples of the molecular dynamics calculation program include gromacs (Groningen Machine for Chemical Simulations), amber (Assisted Model Building with Energy Refinement), charmm, tinker, and lammps.

Examples of the solvent include water.

<Second Step>

The second step is a step for calculating an energy change ($\Delta G_2$) between the bound state ($\lambda=0$) where the compound and the protein are bound, and the unbound state ($\lambda=1$) where the compound and the protein are not bound.

The bound state ($\lambda=0$) means a state where the interaction between the compound and the protein is the strongest.

The unbound state ($\lambda=1$) means a state where there is no interaction between the compound and the protein.

The second step contains at least the following first to fifth processes, and may further contain other processes according to the necessity.

First process: a process for determining a distance ($D_{th}$), within which structure sampling is performed

Second process: a process for calculating a binding energy change ($\Delta G_{21}$) between the compound and the protein which are spaced within a distance equal to or shorter than the distance ($D_{th}$) in a state ($\lambda=\lambda m$) that is between the bound state ($\lambda=0$) and the unbound state ($\lambda=1$), in which a state where a distance between the compound and the protein is equal to or shorter than the distance ($D_{th}$) is present

Third process: a process for calculating a solvation energy change ($\Delta G_{23}$) between the solvent and the compound with ignoring an influence of the protein in the unbound state ($\lambda=1$) and the state ($\lambda=\lambda n$) that can be regarded as the same to the unbound state ($\lambda=1$)

Fourth process: a process for calculating a binding energy change ($\Delta G_{22}$) between the compound and the protein in a state between the state ($\lambda=\lambda m$) and the state ($\lambda=\lambda n$), by interpolating the state ($\lambda=\lambda m$) and the state ($\lambda=\lambda n$)

Fifth process: a process for calculating a correction term ($\Delta G_{24}$) with respect to a standard state, based on a volume of a space calculated from the distance ($D_{th}$)

-First Process-

The first process is appropriately selected depending on the intended purpose without any limitation, provided that the first process is a process for determining a distance ($D_{th}$), within which structure sampling is performed, based on a distance between the compound and the protein in the bound state ($\lambda=0$).

The distance between the compound and the protein is preferably a distance between a center of gravity of the compound, and a center of gravity of a space formed by linking centers of gravity of a plurality of amino acid residues constituting a binding site in the protein.

The distance ($D_{th}$) is preferably selected from distances between 90% and 100% of a minimum value in a frequency distribution of a distance that is a result obtained by simulating the distance between the compound and the protein in the bound state ($\lambda$=0).

One example of a determination method of the distance ($D_{th}$) is explained.

First, a distance between the compound and the protein in the bound state ($\lambda$=0) is plotted with simulation time by a molecular dynamics (MD) method. One example thereof is depicted in FIG. 4. Based on the obtained plot, any of distances that are between 90% and 100% of the minimum value of the distance between the compound and the protein in the frequency distribution is determined as the distance ($D_{th}$). The distance ($D_{th}$) can be always determined as 100%. In this case, however, a scope for which structure sampling is performed becomes unnecessarily wide, if the distance, which is irregularly large during the simulation, is determined as the ($D_{th}$). Accordingly, the distance is preferably selected from the range between 90% and 100% of the minimum value.

-Second Process-

The second process is appropriately selected depending on the intended purpose without any limitation, provided that the second process is a process for calculating a binding energy change ($\Delta G_{21}$) between the compound and the protein within a distance equal to or shorter than the distance ($D_{th}$) on a state ($\lambda=\lambda m$) that is a state between the bound state ($\lambda$=0) and the unbound state ($\lambda$=1), the state ($\lambda=\lambda m$) including a state in which a distance between the compound and the protein is equal to or shorter than the distance ($D_{th}$).

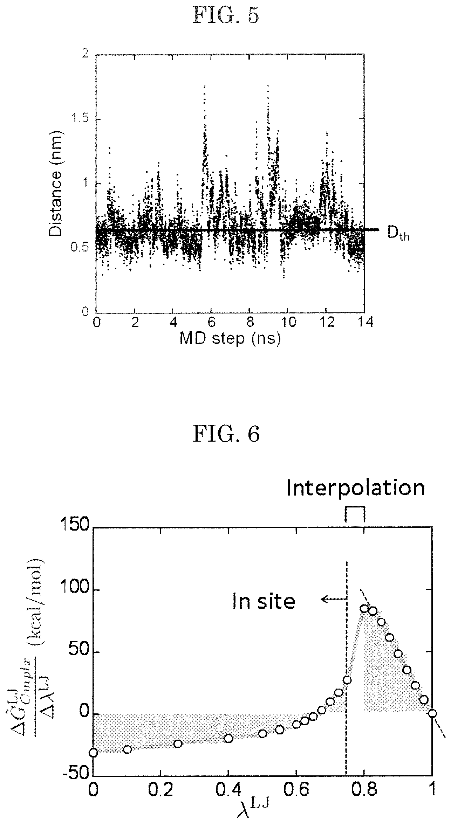

One example of the second process is described below. In the second process, a distance between the compound and the protein in a state between the bound state ($\lambda$=0) and the unbound state ($\lambda$=1) is plotted with simulation time by a molecular dynamics (MD) method. One example thereof is depicted in FIG. 5. In the state ($\lambda=\lambda m$) where the distance between the compound and the protein, which is equal to or shorter than the distance ($D_{th}$), is present as depicted in FIG. 5, structure sampling is performed within the distance equal to or shorter than the distance ($D_{th}$). In FIG. 5, the distance Dth is set to 0.7 nm, and structure sampling is performed when the distance between the compound and the protein is 0.7 nm or shorter.

-Third Process-

The third process is appropriately selected depending on the intended purpose without any limitation, provided that the third process is a process for calculating a change in solvation energy ($\Delta G_{23}$) between the solvent and the compound in the unbound state ($\lambda$=1) and a state ($\lambda=\lambda n$) that can be regarded as the same to the unbound state ($\lambda$=1), with ignoring an influence of the protein.

The state that can be regarded as the same to the unbound state is a state that is a state between the bound state ($\lambda$=0) and the unbound state ($\lambda$=1), and is a state that can be judged as that there is hardly any interaction between the compound and the protein.

Note that, $\lambda m$ and $\lambda n$ satisfy the relation of $0 \leq \lambda m < \lambda n < 1$.

-Fourth Process-

The fourth process is appropriately selected depending on the intended purpose without any limitation, provided that the fourth process is a process for calculating a change in binding energy ($\Delta G_{22}$) between the compound and the protein in a state between the state ($\lambda=\lambda m$) and the state ($\lambda=\lambda n$), with interpolating from the state ($\lambda=\lambda m$)and the state ($\lambda=\lambda n$).

One example of a flow of the second process to the fourth process is explained here.

In this example, explanations are provided by taking a disappearance process of the Lennard-Jones (LJ) interaction illustrated in FIG. 6, as an example.

First, a change ($\Delta G_{21}$) in binding energy between the compound and the protein is calculated with samples whose distance between a compound and a protein is within the distance ($D_{th}$) from the bound state ($\lambda^{LJ}$=0) to the unbound state ($\lambda^{LJ}$=1) based on the distance Dth determined by the first process. As the interaction between the compound and the protein weakens, it becomes a state where there is no sample within the distance ($D_{th}$) (around $\lambda^{LJ}$=0.75 in FIG. 6). The calculation is performed until such the state.

Next, a change ($\Delta G_{23}$) in solvation energy between the solvent and the compound is calculated from the unbound state ($\lambda^{LJ}$=1) to the bound state ($\lambda^{LJ}$=0) until the limit at which it can be judged that there is hardly any interaction between the compound and the protein. In FIG. 6, the calculation was carried out to around $\lambda^{LJ}$=0.8.

Regarding the region (0.75<$\lambda^{LJ}$<0.8) between the regions within which the calculation was performed above,

the energy change ($\Delta G_{22}$) is calculated by interpolating from the results of $\lambda^{LJ}$=0.75 and $\lambda^{LJ}$=0.8.

-Fifth Process-

The fifth process is appropriately selected depending on the intended purpose without any limitation, provided that it is calculating a correction term ($\Delta G_{24}$) with respect to a standard state based on a volume of a space calculated from the distance ($D_{th}$).

One example of the fifth process is explained.

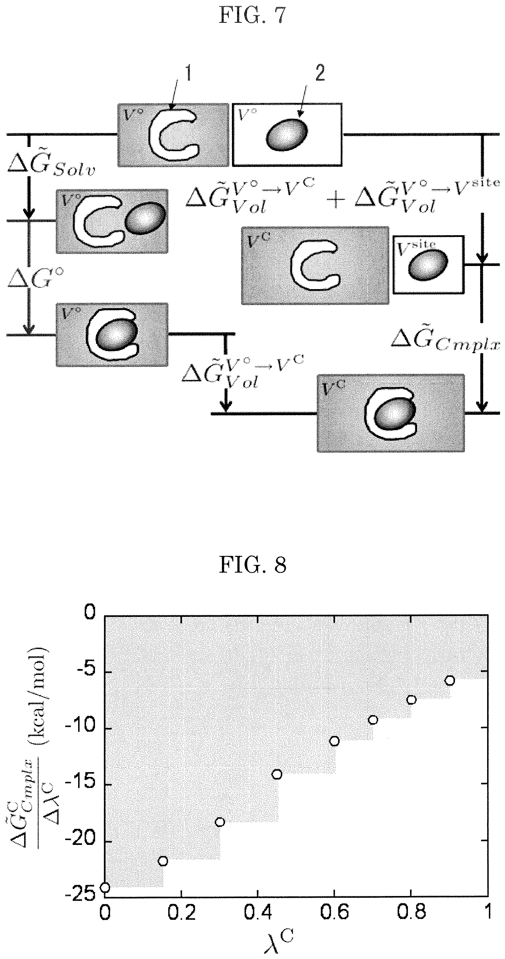

FIG. 7 is a diagram illustrating a change in the state of the compound (2) and the protein (1).

The binding free energy ($\Delta G^\circ$) to be calculated based on the state change of FIG. 7 can be calculated, for example, by the following expressions.

$$\Delta G^\circ = \Delta \tilde{G}_{Cmplx} - \Delta \tilde{G}_{Solv} + \Delta \tilde{G}_{Vol}^{V^\circ \to V^{site}}$$

$$\Delta \tilde{G}_{Vol}^{V^\circ \to V^{site}} = -k_B T \ln \left( \frac{V^{site}}{V^\circ} \right)$$

In FIG. 7 and the expressions above, $V^0$ represents a standard state volume, $V^C$ represents a unit cell volume in a composition of the compound and the protein, and $V^{site}$ represents a volume of a bind pocket of the protein.

Among the two expressions above, the left hand member of the bottom expression corresponds to a correction term ($\Delta G_{24}$).

A calculation program used in the second step is appropriately selected depending on the intended purpose without any limitation, and examples thereof include a molecular dynamics calculation program. Examples of the molecular dynamics calculation program include gromacs.

In the calculation method of binding free energy, it is preferred that the binding free energy be calculated separately for Coulomb interaction and for Lennard-Jones (LJ) interaction, and the binding free energy for the Coulomb interaction be calculated followed by calculating the binding free energy for the Lennard-Jones interaction.

The free energy is state variable, and thus typically it is not influenced by a process. When the Lennard-Jones interaction is made disappeared first, however, atoms come extremely close to each other due to the remained Coulomb interaction (which gives large interaction energy compared to the Lennard-Jones interaction). Therefore, a calculation may be difficult to perform. When the Coulomb interaction is made disappeared first, on the other hand, the Lennard-Jones interaction can be treated as a soft core potential (in order to avoid a case where different atoms are brought extremely close to the same location), and therefore an accurate calculation can be performed.

One example of a disappearance process of the Coulomb interaction between the compound and the protein is depicted in FIG. 8.

(Screening Method of Compound)

The disclosed screening method of a compound contains: calculating binding free energy of a protein with a plurality of compounds in accordance with the disclosed calculation method of binding free energy; and selecting a compound based on the calculated binding free energy.

A method for selecting the plurality of compounds is appropriately selected depending on the intended purpose without any limitation, and examples thereof include a method for selecting the plurality of compounds from a compound library, which is to be a screening target, based on information about attributes of compounds.

The selecting the compound is appropriately selected depending on the intended purpose without any limitation, and examples thereof include selecting a compound having an optimal value amount the calculated values of the binding free energy, as a drug candidate molecule suitable for the protein.

(Program)

The disclosed program is a program that causes a computer to execute at least a first step and a second step.

The program calculates binding free energy between a compound and a protein in a solvent.

The first step and the second step are respectively the first step and the second step of the disclosed calculation method of binding free energy.

The program can be formed with various conventional programming languages depending on a configuration of a computer system for use, and a type or version of an operation system.

The program may be recorded on a recording medium, such as an internal hard disk, and an external hard disk, or may be recorded on a recording medium, such as a compact disc read only memory (CD-ROM), a digital versatile disk read only memory (DVD-ROM), a magneto-optical disk (MO disk), and universal serial bus (USB) flash drive (USB memory). In the case where the program is recorded on a recording medium, such as CD-ROM, DVD-ROM, MO disk, and USB memory, the program can be directly used via a recording device reading device equipped with a computer system, or is used by installing on a hard disk according to the necessity at any time. It is also possible that the program is recorded on an external memory region (e.g., another computer), which can be accessed from a computer system through an information communication network, and the program is directly used from the external storage region via the information communication network, or is used by installing a hard disk according to the necessity at any time.

(Recording Medium Readably by Computer)

The recording medium readable by a computer stores therein the disclosed program.

The recording medium readable by a computer is appropriately selected depending on the intended purpose without any limitation, and examples thereof include an internal hard disk, an external hard disk, CD-ROM, DVD-ROM, a MO disk, and an USB memory.

(Calculation Device of Binding Free Energy)

The disclosed calculation device of binding free energy contains a computer, and the recording medium readable by the computer.

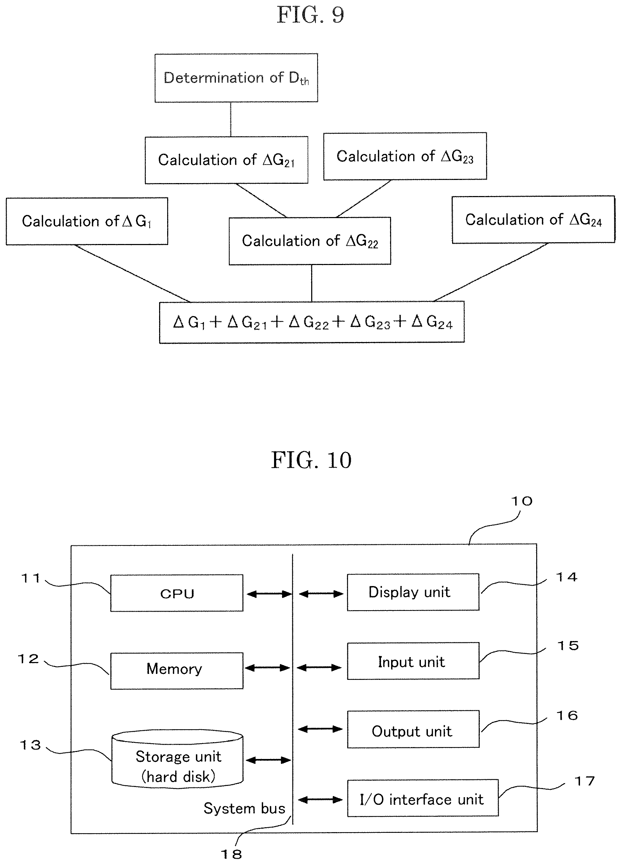

A flow chart of one example of the disclosed calculation method of binding free energy is depicted in FIG. 9.

First, the solvation energy ($\Delta G_1$) between the solvent and the compound is calculated (calculation of AGi).

Next, a distance ($D_{th}$), within which structure sampling is performed, is determined based on a distance between the compound and the protein in the bound state ($\lambda=0$) (determination of $D_{th}$).

Next, a change in binding energy ($\Delta G_{21}$) between the compound and the protein is calculated within a distance equal to or shorter than the distance ($D_{th}$) in a state ($\lambda=\lambda m$) that is a state between the bound state ($\lambda=0$) and the unbound state ($\lambda=1$), the state ($\lambda=\lambda m$) including a state in which a distance between the compound and the protein is equal to or shorter than the distance ($D_{th}$) (calculation of $\Delta G_{21}$).

Next, a change in solvation energy ($\Delta G_{23}$) between the solvent and the compound in the unbound state ($\lambda=1$) and a state ($\lambda=\lambda n$) that can be regarded as the same to the unbound state ($\lambda=1$), is calculated with ignoring an influence of the protein (calculation of $\Delta G_{23}$).

Next, a change in binding energy ($\Delta G_{22}$) between the compound and the protein in a state between the state ($\lambda=\lambda m$) and the state ($\lambda=\lambda n$), is calculated with interpolating from the state ($\lambda=\lambda m$) and the state ($\lambda=\lambda n$) (calculation of $\Delta G_{22}$).

Next, a correction term ($\Delta G_{24}$) with respect to a standard state is calculated based on a volume of a space calculated from the distance ($D_{th}$)

(calculation of $\Delta G_{24}$).

Finally, the sum of $\Delta G_1$, $\Delta G_{21}$, $\Delta G_{22}$, $\Delta G_{23}$, and $\Delta G_{24}$ is obtained.

In the manner as described above, binding free energy between a compound and a protein in a solvent can be calculated.

Note that, as illustrated in FIG. 9, there is no limitation in the order for performing a calculation of $\Delta G_1$, and other processes (determination of Dth, calculation of $\Delta G_{21}$, calculation of $\Delta G_{22}$, calculation of $\Delta G_{23}$, and calculation of $\Delta G_{24}$). Moreover, there is not limitation in the order for performing the calculation of $\Delta G_{24}$, and the calculations of $\Delta G_{21}$, $\Delta G_{22}$, and $\Delta G_{23}$. The calculation of $\Delta G_{22}$ is preferably performed after determining the state ($\lambda=\lambda m$) through the calculation of $\Delta G_{21}$, and after determining the state ($\lambda=\lambda n$) through the calculation of $\Delta G_{23}$.

A structural example of a hardware of the disclosed calculation device of binding free energy is illustrated in FIG. 10.

The calculation device 10 of binding free energy is composed, for example, by connecting a CPU 11, a memory 12, a storage unit 13, a display unit 14, an input unit 15, an output unit 16, an I/O interface unit 17, etc. via a system bus 18.

The CPU (Central Processing Unit) 11 is configured to operate (e.g., four arithmetic operation, and comparison operation), and to control operations of a hardware and a software.

The memory 12 is a memory, such as a random access memory (RAM), and read only memory (ROM). The RAM saves an operation system (OS) and application programs read from the ROM and the storage unit 13, and functions as a main memory and work area of the CPU 11.

The storage unit 13 is a device for storing various programs and data, and examples thereof is a hard dist. In the storage unit 13, a program performed by the CPU 11, data required for carrying out a program, OS, etc. are stored.

The program is stored in the storage unit 13, loaded on RAM (a main memory) of the memory 12, and is carried out by the CPU 11.

The display unit 14 is a display device, and examples thereof include display devices, such as a CRT monitor, and a liquid crystal panel.

The input unit 15 is an input device for various data, and examples thereof include a key board, and a pointing device (e.g., a mouse).

The output unit 16 is an output device of various data, and examples thereof include a printer.

The I/O interface unit 17 is an interface for connecting with various outer devices. For example, the I/O interface unit 17 allows input and output of data of CD-ROM, DVD-ROM, a MO disk, or an USB memory.

The disclosed calculation method of binding free energy can solve the aforementioned various problems in the art, can achieve the aforementioned object, and can provide a method, which accurately calculate binding free energy between a protein and a compound.

The disclosed screening method of a compound can solve the aforementioned various problems in the art, can achieve the aforementioned object, and can provide a method, which efficiently screens a compound.

The disclosed program can solve the aforementioned various problems in the art, can achieve the aforementioned object, and can provide a program, which accurately calculates binding free energy between a protein and a compound.

The disclosed calculation device of binding free energy can solve the aforementioned various problems in the art, can achieve the aforementioned object, and can provide a device, which accurately calculates binding free energy between a protein and a compound.

Example

The disclosed calculation method of binding free energy is explained through Example hereinafter, but Example shall not be construed to as limiting the disclosed calculation method of binding free energy.

Using the disclosed calculation method of binding free energy, binding free energy of a protein and ligands in water was calculated, where [1fkj] and [1fkg] registered in the protein data bank (PDB) were used as the protein.

[1fkj]

Protein: FK506 BINDING PROTEIN
Ligand: 8-DEETHYL-8-[BUT-3-ENYL]-ASCOMYCIN, $C_{44}H_{69}NO_{12}$ (molecular weight: 803)

[1fkg]

Protein: FK506 BINDING PROTEIN
Ligand: 1,3-DIPHENYL-1-PROPYL-1-(3,3-DIMETHYL-1,2-DIOXYPENTYL)-2-PIPERIDINE CARBOXYLATE, $C_{28}H_{35}NO_4$ (molecular weight: 449)

In the calculation below, gromacs (Groningen Machine for Chemical Simulations), which was a molecular dynamics calculation program, was used.

As for a distance between the compound and the protein when determining a distance ($D_{th}$) within which structure sampling would be performed, used was a distance from a center of gravity of the compound, and a center of gravity of a space formed by linking centers of gravity of a plurality of amino acid residues constituting a binding site in the protein.

The distance Dth, within which structure sampling would be performed, was determined in the following manner.

A distance between the compound and the protein in the bound state ($\lambda$=0) was plotted with simulation time using gromacs. The distance, which was 95% from the minimum value of the distance between the compound and the protein in the frequency distribution based on the obtained plot, was determined as the ($D_{th}$). The specific distance ($D_{th}$) was 0.77 nm in the calculation of [1fkj], and was 0.85 nm in the calculation of [1fkg].

In accordance with the disclosed method, $\Delta G_1$, and $\Delta G_2$ ($\Delta G_{21}$, $\Delta G_{22}$, $\Delta G_{23}$, $\Delta G_{24}$) were calculated.

Note that, the maximum value of $\lambda = \lambda m$ at the time of calculating $\Delta G_{21}$ was determined by setting the maximum value of $\lambda$ with which a structure containing the compound in the distance $D_{th}$ did not appear, as $\lambda m$. Moreover, the minimum value at the time of calculating $\Delta G_{23}$ is determined by setting the minimum value of $\lambda$ with which it started to match with a change of the free energy of the compound in the solvent, as $\lambda n$.

Note that, the calculation was performed separately for the Coulomb interaction and for the Lennard-Jones interaction. The binding free energy was first calculated for the Coulomb interaction, and then calculated for the Lennard-Jones interaction. As a result, the Lennard-Jones interaction could be treated as a soft core potential, and therefore an accurate calculation was possible.

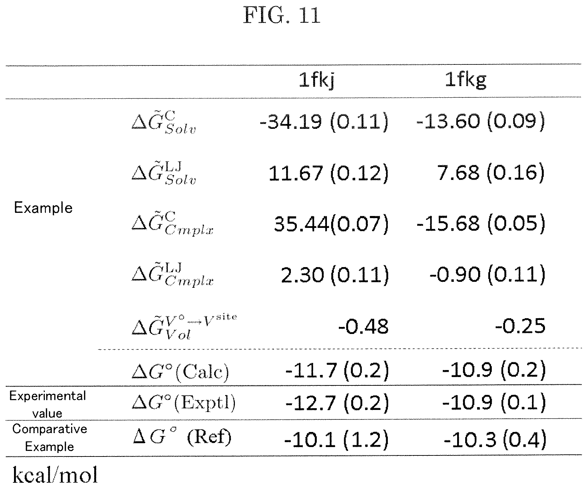

The calculation results are depicted in FIG. 11.

Example in FIG. 11 is the results of Example. The experimental value is the experimental value disclosed in Holt et al., J. Am. Chem. Soc. 1993, 115, 9925-9938. Comparative Example is the calculation value disclosed in Wang et al., Biophysical Journal Vol. 91, 2798-2814, and is the calculation value in the case where the restriction with a spring is performed.

The calculation result (-10.9 kcal/mol) of 1fkg of Example is matched to the experimental value (-10.9 kcal/mol), and is more excellent than the calculation result (-10.3 kcal/mol) of Comparative Example.

The calculation result (-11.7 kcal/mol) of 1fkj of Example is a value slightly different from the experimental value (-12.7 kcal/mol), but is a value closer to the experimental value than Comparative Example, (-10.1 kcal/mol). Comparative Example of 1fkj and the experimental value of 1fkj are largely different from each other, probably because a molecule of the ligand is large. In Example, the calculation result with relatively high accuracy could be attained even when the ligand molecule was large.

**Claims**

1. A method of calculating, using a computer, the binding free energy between a compound and a protein in a solvent, the method comprising:

   calculating solvation energy ($\Delta G_1$) between the solvent and the compound; and
   calculating an energy change ($\Delta G_2$) between a bound state ($\lambda = 0$) where the compound and the protein are bound, and an unbound state ($\lambda = 1$) where the compound and the protein are not bound,
   wherein the binding free energy is the sum of the solvation energy ($\Delta G_1$) between the solvent and the compound and the energy change ($\Delta G_2$) between the bound state ($\lambda = 0$), and the unbound state ($\lambda = 1$), and
   wherein the calculating the energy change ($\Delta G_2$) comprises:

   determining a distance ($D_{th}$), within which structure sampling is performed, based on a distance between the compound and the protein in the bound state ($\lambda = 0$), wherein the distance ($D_{th}$) is selected from distances between 90% and 100% of a minimum value in a frequency distribution of a distance that is a result obtained by simulating the distance between the compound and the protein in the bound state ($X = 0$);
   calculating a change in binding energy ($\Delta G_{21}$) between the compound and the protein within a distance equal to or shorter than the distance ($D_{th}$) on a state ($\lambda = \lambda m$) that is a state between the bound state ($\lambda = 0$) and the unbound state ($\lambda = 1$), the state ($\lambda = \lambda m$) including a state in which a distance between the compound and the protein is equal to or shorter than the distance ($D_{th}$),
   calculating a change in solvation energy ($\Delta G_{23}$) between the solvent and the compound in the unbound state ($\lambda = 1$) and a state ($\lambda = \lambda n$) that can be regarded as the same as the unbound state ($\lambda = 1$), the state ($\lambda = \lambda n$) that can be regarded as the same as the unbound state ($\lambda = 1$) being a state between the bound state ($\lambda = 0$) and the unbound state ($\lambda = 1$) wherein there is hardly any interaction between the compound and the protein,
   calculating a change in binding energy ($\Delta G_{22}$) between the compound and the protein on a state between the state ($\lambda = \lambda m$) and the state ($\lambda = \lambda n$), with interpolating from the state ($\lambda = \lambda m$) and the state ($\lambda = \lambda n$), and
   calculating a correction term ($\Delta G_{24}$) with respect to a standard state of the compound, based on a volume of a space calculated from the distance ($D_{th}$), the volume being a spherical volume having a radius of the distance ($D_{th}$), the correction term ($\Delta G_{24}$) being calculated according to the formula

$$\Delta G_{24} = -k_B T \left( \frac{V^{site}}{V^{\circ}} \right)$$

where $V^0$ represents a standard state volume, $V^{site}$ represents a volume of a bind pocket of the protein, T is temperature and $k_B$ is the Boltzmann constant,

wherein the energy change ($\Delta G_2$) is the sum of: the change in binding energy ($\Delta G_{21}$) between the compound and the protein within a distance equal to or shorter than the distance ($D_{th}$) on a state ($\lambda=\lambda m$), the change in binding energy ($\Delta G_{22}$) between the compound and the protein on a state between the state ($\lambda=\lambda m$) and the state ($\lambda=\lambda n$), the change in solvation energy ($\Delta G_{23}$) between the solvent and the compound in the unbound state ($\lambda=1$) and a state ($\lambda=\lambda n$), and the correction term ($\Delta G_{24}$) with respect to the standard state of the compound.

2. The method according to claim 1, wherein the solvent is water.

3. The method according to any of claims 1 or 2, wherein the distance between the compound and the protein is a distance between a center of gravity of the compound, and a center of gravity of a space formed by linking centers of gravity of a plurality of amino acid residues constituting a binding site in the protein.

4. The method according to any of claims 1 to 3, wherein the binding free energy is calculated separately for Coulomb interaction and for Lennard-Jones interaction, and the binding free energy is calculated for the Coulomb interaction, followed by calculating for the Lennard-Jones interaction.

5. A screening method for a compound, comprising:

calculating binding free energies of a protein with each of a plurality of compounds in accordance with the calculation method of binding free energy according to any of claims 1 to 4; and
selecting the compound based on the calculated binding free energies, wherein the selected compound is a compound having an optimal value, from among the calculated binding free energies of the plurality of compounds, as a drug candidate molecule suitable for the protein.

6. A program for causing a computer to execute calculation of the binding free energy between a compound and a protein in a solvent by:

calculating solvation energy ($\Delta G_1$) between the solvent and the compound; and
calculating an energy change ($\Delta G_2$) between a bound state ($\lambda=0$) where the compound and the protein are bound, and an unbound state ($\lambda=1$) where the compound and the protein are not bound,
wherein the binding free energy is the sum of the solvation energy ($\Delta G_1$) between the solvent and the compound and the energy change ($\Delta G_2$) between the bound state ($\lambda=0$), and the unbound state ($\lambda=1$), and
wherein the calculating the energy change ($\Delta G_2$) comprises:

determining a distance ($D_{th}$), within which structure sampling is performed, based on a distance between the compound and the protein in the bound state ($\lambda=0$), wherein the distance ($D_{th}$) is selected from distances between 90% and 100% of a minimum value in a frequency distribution of a distance that is a result obtained by simulating the distance between the compound and the protein in the bound state ($\lambda=0$);
calculating a change in binding energy ($\Delta G_{21}$) between the compound and the protein within a distance equal to or shorter than the distance ($D_{th}$) on a state ($\lambda=\lambda m$) that is a state between the bound state ($\lambda=0$) and the unbound state ($\lambda=1$), the state ($\lambda=\lambda m$) including a state in which a distance between the compound and the protein is equal to or shorter than the distance ($D_{th}$),
calculating a change in solvation energy ($\Delta G_{23}$) between the solvent and the compound in the unbound state ($\lambda=1$) and a state ($\lambda=\lambda n$) that can be regarded as the same as the unbound state ($\lambda=1$), the state ($\lambda=\lambda n$) that can be regarded as the same as the unbound state ($\lambda=1$) being a state between the bound state ($\lambda=0$) and the unbound state ($\lambda=1$) wherein there is hardly any interaction between the compound and the protein,
calculating a change in binding energy ($\Delta G_{22}$) between the compound and the protein on a state between the state ($\lambda=\lambda m$) and the state ($\lambda=\lambda n$), with interpolating from the state ($\lambda=\lambda m$) and the state ($\lambda=\lambda n$), and
calculating a correction term ($\Delta G_{24}$) with respect to a standard state of the compound, based on a volume of a space calculated from the distance ($D_{th}$), the volume being a spherical volume having a radius of the distance ($D_{th}$), the correction term ($\Delta G_{24}$) being calculated according to the formula

$$\Delta G_{24} = -k_B T \left( \frac{V^{site}}{V^\circ} \right)$$

where $V^0$ represents a standard state volume, $V^{site}$ represents a volume of a bind pocket of the protein, T is temperature and $k_B$ is the Boltzmann constant,

wherein the energy change ($\Delta G_2$) is the sum of: the change in binding energy ($\Delta G_{21}$) between the compound and the protein within a distance equal to or shorter than the distance ($D_{th}$) on a state ($\lambda=\lambda m$), the change in binding energy ($\Delta G_{22}$) between the compound and the protein on a state between the state ($\lambda=\lambda m$) and the state ($\lambda=\lambda n$), the change in solvation energy ($\Delta G_{23}$) between the solvent and the compound in the unbound state ($\lambda=1$) and a state ($\lambda=\lambda n$), and the correction term ($\Delta G_{24}$) with respect to the standard state of the compound.

7. The program according to claim 6, wherein the solvent is water.

8. The program according to any of claims 6 or 7, wherein the distance between the compound and the protein is a distance between a center of gravity of the compound, and a center of gravity of a space formed by linking centers of gravity of a plurality of amino acid residues constituting a binding site in the protein.

9. The program according to any of claims 6 to 8, wherein the binding free energy is calculated separately for Coulomb interaction and for Lennard-Jones interaction, and the binding free energy is calculated for the Coulomb interaction, followed by calculating for the Lennard-Jones interaction.

10. A device for calculation of binding free energy, comprising:

a computer; and
a recording medium readable by the computer, where the recording medium is configured to store therein a program which, if executed by a computer, causes the computer to execute:

calculating solvation energy ($\Delta G_1$) between the solvent and the compound; and
calculating an energy change ($\Delta G_2$) between a bound state ($\lambda=0$) where the compound and the protein are bound, and an unbound state ($\lambda=1$) where the compound and the protein are not bound,

wherein the binding free energy is the sum of the solvation energy ($\Delta G_1$) between the solvent and the compound and the energy change ($\Delta G_2$) between the bound state ($\lambda=0$), and the unbound state ($\lambda=1$), and
wherein the calculating the energy change ($\Delta G_2$) comprises:

determining a distance ($D_{th}$), within which structure sampling is performed, based on a distance between the compound and the protein in the bound state ($\lambda=0$), wherein the distance ($D_{th}$) is selected from distances between 90% and 100% of a minimum value in a frequency distribution of a distance that is a result obtained by simulating the distance between the compound and the protein in the bound state ($\lambda=0$);
calculating a change in binding energy ($\Delta G_{21}$) between the compound and the protein within a distance equal to or shorter than the distance ($D_{th}$) on a state ($\lambda=\lambda m$) that is a state between the bound state ($\lambda=0$) and the unbound state ($\lambda=1$), the state ($\lambda=\lambda m$) including a state in which a distance between the compound and the protein is equal to or shorter than the distance ($D_{th}$),
calculating a change in solvation energy ($\Delta G_{23}$) between the solvent and the compound in the unbound state ($\lambda=1$) and a state ($\lambda=\lambda n$) that can be regarded as the same as the unbound state ($\lambda=1$), the state ($\lambda=\lambda n$) that can be regarded as the same as the unbound state ($\lambda=1$) being a state between the bound state ($\lambda=0$) and the unbound state ($\lambda=1$) wherein there is hardly any interaction between the compound and the protein,
calculating a change in binding energy ($\Delta G_{22}$) between the compound and the protein on a state between the state ($\lambda=\lambda m$) and the state ($\lambda=\lambda n$), with interpolating from the state ($\lambda=\lambda m$) and the state ($\lambda=\lambda n$), and
calculating a correction term ($\Delta G_{24}$) with respect to a standard state of the compound, based on a volume of a space calculated from the distance ($D_{th}$), the volume being a spherical volume having a radius of the distance ($D_{th}$), the correction term ($\Delta G_{24}$) being calculated according to the formula

$$\Delta G_{24} = -k_B T \left( \frac{V^{site}}{V^{\circ}} \right)$$

where $V^0$ represents a standard state volume, $V^{site}$ represents a volume of a bind pocket of the protein, T is temperature and $k_B$ is the Boltzmann constant,

wherein the energy change ($\Delta G_2$) is the sum of: the change in binding energy ($\Delta G_{21}$) between the compound and the protein within a distance equal to or shorter than the distance ($D_{th}$) on a state ($\lambda=m$), the change in binding energy ($\Delta G_{22}$) between the compound and the protein on a state between the state ($\lambda=\lambda m$) and the state ($\lambda=\lambda n$), the change in solvation energy ($\Delta G_{23}$) between the solvent and the compound in the unbound state ($\lambda=1$) and a state ($\lambda=\lambda n$), and the correction term ($\Delta G_{24}$) with respect to the standard state of the compound.

11. The device according to claim 10, wherein the solvent is water.

12. The device according to any of claims 10 or 11, wherein the distance between the compound and the protein is a distance between a center of gravity of the compound, and a center of gravity of a space formed by linking centers of gravity of a plurality of amino acid residues constituting a binding site in the protein.

**Patentansprüche**

1. Verfahren zur Berechnung der freien Bindungsenergie zwischen einer Verbindung und einem Protein in einem Lösungsmittel unter Verwendung eines Computers, wobei das Verfahren umfasst:

Berechnen einer Solvatationsenergie ($\Delta G_1$) zwischen dem Lösungsmittel und der Verbindung; und
Berechnen einer Energieänderung ($\Delta G_2$) zwischen einem gebundenen Zustand ($\lambda=0$), in dem die Verbindung und das Protein gebunden sind, und einem ungebundenen Zustand ($\lambda=1$), in dem die Verbindung und das Protein nicht gebunden sind,
wobei die freie Bindungsenergie die Summe der Solvatationsenergie ($\Delta G_1$) zwischen dem Lösungsmittel und der Verbindung und der Energieänderung ($\Delta G_2$) zwischen dem gebundenen Zustand ($\lambda=0$) und dem ungebundenen Zustand ($\lambda=1$) ist, und
wobei das Berechnen der Energieänderung ($\Delta G_2$) umfasst:

Bestimmen eines Abstands ($D_{th}$), innerhalb dem eine strukturelle Probennahme durchgeführt wird, basierend auf einem Abstand zwischen der Verbindung und dem Protein im gebundenen Zustand ($\lambda=0$); wobei der Abstand ($D_{th}$) aus Abständen zwischen 90% und 100% eines Minimalwerts in einer Häufigkeitsverteilung eines Abstands ausgewählt wird, der ein Ergebnis ist, das durch Simulieren des Abstands zwischen der Verbindung und dem Protein im gebundenen Zustand ($\lambda=0$) erhalten wird;
Berechnen einer Änderung in der Bindungsenergie ($\Delta G_{21}$) zwischen der Verbindung und dem Protein in einem Abstand, der gleich oder kürzer als der Abstand ($D_{th}$) in einem Zustand ($\lambda=\lambda m$) ist, der ein Zustand zwischen dem gebundenen Zustand ($\lambda=0$) und dem ungebundene Zustand ($\lambda=1$) ist, wobei der Zustand ($\lambda=\lambda m$) einen Zustand aufweist, in dem ein Abstand zwischen der Verbindung und dem Protein gleich oder kürzer als der Abstand ($D_{th}$) ist,
Berechnen einer Änderung in der Solvatationsenergie ($\Delta G_{23}$) zwischen dem Lösungsmittel und der Verbindung in dem ungebundenen Zustand ($\lambda=1$) und einem Zustand ($\lambda=\lambda n$), der als der gleiche wie der ungebundene Zustand ($\lambda=1$) angesehen werden kann, wobei der Zustand ($\lambda=\lambda n$), der als der gleiche wie der ungebundene Zustand ($\lambda=1$) angesehen werden kann, ein Zustand zwischen dem gebundenen Zustand ($\lambda=0$) und dem ungebundenen Zustand ($\lambda=1$) ist, zwischen dem kaum eine Wechselwirkung der Verbindung und dem Protein besteht,
Berechnen einer Änderung in der Bindungsenergie ($\Delta G_{22}$) zwischen der Verbindung und dem Protein in einem Zustand zwischen dem Zustand ($\lambda=\lambda m$) und dem Zustand ($\lambda=\lambda n$), mit einer Interpolation des Zustands ($\lambda=\lambda m$) und des Zustands ($\lambda=\lambda n$), und
Berechnen eines Korrekturterms ($\Delta G_{24}$) in Bezug auf einen Standardzustand der Verbindung basierend auf einem aus dem Abstand ($D_{th}$) berechneten Volumen eines Raumes, wobei das Volumen ein kugelförmiges Volumen mit einem Radius des Abstands ($D_{th}$) ist; wobei der Korrekturterm ($\Delta G_{24}$) berechnet wird nach der Formel

$$\Delta G_{24} = -k_B T \left( \frac{V^{site}}{V^\circ} \right)$$

wobei $V^0$ ein Standardzustandsvolumen darstellt, $V^{site}$ ein Volumen einer Bindungstasche des Proteins darstellt, T die Temperatur ist, und $k_B$ die Boltzmann-Konstante ist,

wobei die Energieänderung ($\Delta G_2$) die Summe ist aus: der Änderung in der Bindungsenergie ($\Delta G_{21}$) zwischen der Verbindung und dem Protein in einem Abstand, der gleich oder kürzer als der Abstand ($D_{th}$) in einem Zustand ($\lambda=\lambda$ m) ist, der Änderung in der Bindungsenergie ($\Delta G_{22}$) zwischen der Verbindung und dem Protein in einem Zustand zwischen dem Zustand ($\lambda=Am$) und dem Zustand ($\lambda=\lambda n$), der Änderung der Solvatationsenergie ($\Delta G_{23}$) zwischen dem Lösungsmittel und der Verbindung im ungebundenen Zustand ($\lambda=1$) und einem Zustand ($\lambda=\lambda n$), und dem Korrekturterm ($\Delta G_{24}$) in Bezug auf den Standardzustand der Verbindung.

2. Verfahren nach Anspruch 1, wobei das Lösungsmittel Wasser ist.

3. Verfahren nach einem der Ansprüche 1 oder 2, wobei der Abstand zwischen der Verbindung und dem Protein ein Abstand zwischen einem Schwerpunkt der Verbindung und einem Schwerpunkt eines Raumes ist, der durch Verbinden der Schwerpunkte mehrerer Aminosäurereste, die eine Bindungsstelle in dem Protein bilden, gebildet wird.

4. Verfahren nach einem der Ansprüche 1 bis 3, wobei die freie Bindungsenergie für die Coulomb-Wechselwirkung und für die Lennard-Jones-Wechselwirkung getrennt berechnet wird, und die freie Bindungsenergie für die Coulomb-Wechselwirkung berechnet wird, gefolgt von einer Berechnung für die Lennard-Jones-Wechselwirkung.

5. Screening-Verfahren für eine Verbindung, umfassend:

Berechnen der freien Bindungsenergien eines Proteins mit jeder von mehreren Verbindungen gemäß dem Berechnungsverfahren der freien Bindungsenergie nach einem der Ansprüche 1 bis 4; und
Auswählen der Verbindung basierend auf den berechneten freien Bindungsenergien, wobei die ausgewählte Verbindung eine Verbindung mit einem optimalen Wert ist, aus den berechneten freien Bindungsenergien der mehreren Verbindungen als für das Protein geeignetes Arzneimittelkandidatenmolekül.

6. Programm, das einen Computer veranlasst, eine Berechnung der freien Bindungsenergie zwischen einer Verbindung und einem Protein in einem Lösungsmittel durchzuführen, durch:

Berechnen einer Solvatationsenergie ($\Delta G_1$) zwischen dem Lösungsmittel und der Verbindung; und
Berechnen einer Energieänderung ($\Delta G_2$) zwischen einem gebundenen Zustand ($\lambda=0$), in dem die Verbindung und das Protein gebunden sind, und einem ungebundenen Zustand ($\lambda=1$), in dem die Verbindung und das Protein nicht gebunden sind,
wobei die freie Bindungsenergie die Summe der Solvatationsenergie ($\Delta G_1$) zwischen dem Lösungsmittel und der Verbindung und der Energieänderung ($\Delta G_2$) zwischen dem gebundenen Zustand ($\lambda=0$) und dem ungebundenen Zustand ($\lambda=1$) ist, und
wobei das Berechnen der Energieänderung ($\Delta G_2$) umfasst:

Bestimmen eines Abstands ($D_{th}$), innerhalb dem eine strukturelle Probennahme durchgeführt wird, basierend auf einem Abstand zwischen der Verbindung und dem Protein im gebundenen Zustand ($\lambda=0$); wobei der Abstand ($D_{th}$) aus Abständen zwischen 90% und 100% eines Minimalwerts in einer Häufigkeitsverteilung eines Abstands ausgewählt wird, der ein Ergebnis ist, das durch Simulieren des Abstands zwischen der Verbindung und dem Protein im gebundenen Zustand ($\lambda=0$) erhalten wird;
Berechnen einer Änderung in der Bindungsenergie ($\Delta G_{21}$) zwischen der Verbindung und dem Protein in einem Abstand, der gleich oder kürzer als der Abstand ($D_{th}$) in einem Zustand ($\lambda=\lambda m$) ist, der ein Zustand zwischen dem gebundenen Zustand ($\lambda=0$) und dem ungebundene Zustand ($\lambda=1$) ist, wobei der Zustand ($\lambda=\lambda m$) einen Zustand aufweist, in dem ein Abstand zwischen der Verbindung und dem Protein gleich oder kürzer als der Abstand ($D_{th}$) ist,
Berechnen einer Änderung in der Solvatationsenergie ($\Delta G_{23}$) zwischen dem Lösungsmittel und der Verbindung in dem ungebundenen Zustand ($\lambda=1$) und einem Zustand ($\lambda=\lambda n$), der als der gleiche wie der ungebundene Zustand ($\lambda=1$) angesehen werden kann, wobei der Zustand ($\lambda=\lambda n$), der als der gleiche wie der ungebundene Zustand ($\lambda=1$) angesehen werden kann, ein Zustand zwischen dem gebundenen Zustand (λ=0) und dem ungebundenen Zustand (λ=1) ist, zwischen dem kaum eine Wechselwirkung der Verbindung und dem Protein besteht,

Berechnen einer Änderung in der Bindungsenergie ($\Delta G_{22}$) zwischen der Verbindung und dem Protein in einem Zustand zwischen dem Zustand (λ=λm) und dem Zustand (λ=λn), mit einer Interpolation des Zustands (λ=λm) und des Zustands (λ=λn), und

Berechnen eines Korrekturterms ($\Delta G_{24}$) in Bezug auf einen Standardzustand der Verbindung basierend auf einem aus dem Abstand ($D_{th}$) berechneten Volumen eines Raumes, wobei das Volumen ein kugelförmiges Volumen mit einem Radius des Abstands ($D_{th}$) ist; wobei der Korrekturterm ($\Delta G_{24}$) berechnet wird nach der Formel

$$\Delta G_{24} = -k_B T \left( \frac{V^{site}}{V^0} \right)$$

wobei $V^0$ ein Standardzustandsvolumen darstellt, $V^{site}$ ein Volumen einer Bindungstasche des Proteins darstellt, T die Temperatur ist, und $k_B$ die Boltzmann-Konstante ist,

wobei die Energieänderung ($\Delta G_2$) die Summe ist aus: der Änderung in der Bindungsenergie ($\Delta G_{21}$) zwischen der Verbindung und dem Protein in einem Abstand, der gleich oder kürzer als der Abstand ($D_{th}$) in einem Zustand (λ=λm) ist, der Änderung in der Bindungsenergie ($\Delta G_{22}$) zwischen der Verbindung und dem Protein in einem Zustand zwischen dem Zustand (λ=λm) und dem Zustand (λ=λn), der Änderung der Solvatationsenergie ($\Delta G_{23}$) zwischen dem Lösungsmittel und der Verbindung im ungebundenen Zustand (λ=1) und einem Zustand (λ=λn), und dem Korrekturterm ($\Delta G_{24}$) in Bezug auf den Standardzustand der Verbindung.

7.  Programm nach Anspruch 6, wobei das Lösungsmittel Wasser ist.

8.  Programm nach einem der Ansprüche 6 oder 7, wobei der Abstand zwischen der Verbindung und dem Protein ein Abstand zwischen einem Schwerpunkt der Verbindung und einem Schwerpunkt eines Raumes ist, der durch Verbinden der Schwerpunkte mehrerer Aminosäurereste, die eine Bindungsstelle in dem Protein bilden, gebildet wird.

9.  Programm nach einem der Ansprüche 6 bis 8, wobei die freie Bindungsenergie für die Coulomb-Wechselwirkung und für die Lennard-Jones-Wechselwirkung getrennt berechnet wird, und die freie Bindungsenergie für die Coulomb-Wechselwirkung berechnet wird, gefolgt von einer Berechnung für die Lennard-Jones-Wechselwirkung.

10. Vorrichtung zur Berechnen einer freien Bindungsenergie, umfassend:

einen Computer; und
ein von dem Computer lesbares Aufzeichnungsmedium, wobei das Aufzeichnungsmedium so ausgelegt ist, dass es ein Programm darin speichert, das, wenn es von einem Computer ausgeführt wird, den Computers dazu veranlasst, auszuführen:

Berechnen einer Solvatationsenergie ($\Delta G_1$) zwischen dem Lösungsmittel und der Verbindung; und
Berechnen einer Energieänderung ($\Delta G_2$) zwischen einem gebundenen Zustand (λ=0), in dem die Verbindung und das Protein gebunden sind, und einem ungebundenen Zustand (λ=1), in dem die Verbindung und das Protein nicht gebunden sind,

wobei die freie Bindungsenergie die Summe der Solvatationsenergie ($\Delta G_1$) zwischen dem Lösungsmittel und der Verbindung und der Energieänderung ($\Delta G_2$) zwischen dem gebundenen Zustand (λ=0) und dem ungebundenen Zustand (λ=1) ist, und
wobei das Berechnen der Energieänderung ($\Delta G_2$) umfasst:

Bestimmen eines Abstands ($D_{th}$), innerhalb dem eine strukturelle Probennahme durchgeführt wird, basierend auf einem Abstand zwischen der Verbindung und dem Protein im gebundenen Zustand (λ=0); wobei der Abstand ($D_{th}$) aus Abständen zwischen 90% und 100% eines Minimalwerts in einer Häufigkeitsverteilung eines Abstands ausgewählt wird, der ein Ergebnis ist, das durch Simulieren des Abstands zwischen der Verbindung und dem Protein im gebundenen Zustand (λ=0) erhalten wird;
Berechnen einer Änderung in der Bindungsenergie ($\Delta G_{21}$) zwischen der Verbindung und dem Protein in einem Abstand, der gleich oder kürzer als der Abstand ($D_{th}$) in einem Zustand (λ=λm) ist, der ein Zustand zwischen dem gebundenen Zustand ($\lambda$=0) und dem ungebundene Zustand ($\lambda$=1) ist, wobei der Zustand ($\lambda$=$\lambda$m) einen Zustand aufweist, in dem ein Abstand zwischen der Verbindung und dem Protein gleich oder kürzer als der Abstand ($D_{th}$) ist,

Berechnen einer Änderung in der Solvatationsenergie ($\Delta G_{23}$) zwischen dem Lösungsmittel und der Verbindung in dem ungebundenen Zustand ($\lambda$=1) und einem Zustand ($\lambda$=$\lambda$n), der als der gleiche wie der ungebundene Zustand ($\lambda$=1) angesehen werden kann, wobei der Zustand ($\lambda$=$\lambda$n), der als der gleiche wie der ungebundene Zustand ($\lambda$=1) angesehen werden kann, ein Zustand zwischen dem gebundenen Zustand ($\lambda$=0) und dem ungebundenen Zustand ($\lambda$=1) ist, zwischen dem kaum eine Wechselwirkung der Verbindung und dem Protein besteht,

Berechnen einer Änderung in der Bindungsenergie ($\Delta G_{22}$) zwischen der Verbindung und dem Protein in einem Zustand zwischen dem Zustand ($\lambda$=$\lambda$m) und dem Zustand ($\lambda$=$\lambda$n), mit einer Interpolation des Zustands ($\lambda$=$\lambda$m) und des Zustands ($\lambda$=$\lambda$n), und

Berechnen eines Korrekturterms ($\Delta G_{24}$) in Bezug auf einen Standardzustand der Verbindung basierend auf einem aus dem Abstand ($D_{th}$) berechneten Volumen eines Raumes, wobei das Volumen ein kugelförmiges Volumen mit einem Radius des Abstands ($D_{th}$) ist; wobei der Korrekturterm ($\Delta G_{24}$) berechnet wird nach der Formel

$$\Delta G_{24} = -k_B T \left( \frac{V^{site}}{V^0} \right)$$

wobei $V^0$ ein Standardzustandsvolumen darstellt, $V^{site}$ ein Volumen einer Bindungstasche des Proteins darstellt, T die Temperatur ist, und $k_B$ die Boltzmann-Konstante ist,

wobei die Energieänderung ($\Delta G_2$) die Summe ist aus: der Änderung in der Bindungsenergie ($\Delta G_{21}$) zwischen der Verbindung und dem Protein in einem Abstand, der gleich oder kürzer als der Abstand ($D_{th}$) in einem Zustand ($\lambda$=$\lambda$m) ist, der Änderung in der Bindungsenergie ($\Delta G_{22}$) zwischen der Verbindung und dem Protein in einem Zustand zwischen dem Zustand ($\lambda$=$\lambda$m) und dem Zustand ($\lambda$=$\lambda$n), der Änderung der Solvatationsenergie ($\Delta G_{23}$) zwischen dem Lösungsmittel und der Verbindung im ungebundenen Zustand ($\lambda$=1) und einem Zustand ($\lambda$=$\lambda$n), und dem Korrekturterm ($\Delta G_{24}$) in Bezug auf den Standardzustand der Verbindung.

**11.** Vorrichtung nach Anspruch 10, wobei das Lösungsmittel Wasser ist.

**12.** Vorrichtung nach einem der Ansprüche 10 oder 11, wobei der Abstand zwischen der Verbindung und dem Protein ein Abstand zwischen einem Schwerpunkt der Verbindung und einem Schwerpunkt eines Raumes ist, der durch Verbinden der Schwerpunkte mehrerer Aminosäurereste, die eine Bindungsstelle in dem Protein bilden, gebildet wird.

## Revendications

**1.** Procédé de calcul, au moyen d'un ordinateur, de l'énergie libre de liaison entre un composé et une protéine dans un solvant, le procédé comprenant les étapes ci-dessous consistant à :

calculer une énergie de solvatation ($\Delta G_1$) entre le solvant et le composé ; et
calculer une variation d'énergie ($\Delta G_2$) entre un état lié ($\lambda$=0), où le composé et la protéine sont liés, et un état non lié ($\lambda$=1), où le composé et la protéine ne sont pas liés ;
dans lequel l'énergie libre de liaison est la somme de l'énergie de solvatation ($\Delta G_1$) entre le solvant et le composé et de la variation d'énergie ($\Delta G_2$) entre l'état lié ($\lambda$=0) et l'état non lié ($\lambda$=1) ; et
dans lequel l'étape de calcul de la variation d'énergie ($\Delta G_2$) comprend les étapes ci-dessous consistant à :

déterminer une distance ($D_{th}$) , dans laquelle un échantillonnage de structure est mis en œuvre, sur la base d'une distance entre le composé et la protéine à l'état lié ($\lambda$=0), dans lequel la distance ($D_{th}$) est sélectionnée parmi des distances comprises entre 90 % et 100 % d'une valeur minimale dans une distribution de fréquences d'une distance qui correspond à un résultat obtenu en simulant la distance entre le composé et la protéine à l'état lié ($\lambda$=0) ;
calculer une variation de l'énergie de liaison ($\Delta G_{21}$) entre le composé et la protéine sur une distance égale ou inférieure à la distance ($D_{th}$) à un état ($\lambda$=$\lambda$m) qui correspond à un état compris entre l'état lié ($\lambda$=0) et

l'état non lié (λ=1), l'état (À=Àm) incluant un état dans lequel une distance entre le composé et la protéine est égale ou inférieure à la distance (D_{th}) ;

calculer une variation de l'énergie de solvatation (ΔG_{23}) entre le solvant et le composé à l'état non lié (λ=1) et un état (λ=λn) qui peut être considéré comme identique à l'état non lié (λ=1), l'état (λ=λn) qui peut être considéré comme identique à l'état non lié (λ=1) étant un état compris entre l'état lié (λ=0) et l'état non lié (λ=1) dans lequel il n'existe pratiquement pas d'interaction entre le composé et la protéine ;

calculer une variation de l'énergie de liaison (ΔG_{22}) entre le composé et la protéine à un état compris entre l'état (À=Àm) et l'état (λ=λn), avec une interpolation à partir de l'état (À=Àm) et de l'état (λ=λn) ; et

calculer un terme de correction (ΔG_{24}) par rapport à un état standard du composé, sur la base d'un volume d'un espace calculé à partir de la distance (D_{th}), le volume étant un volume sphérique présentant un rayon de la distance (D_{th}), le terme de correction (ΔG_{24}) étant calculé selon la formule ci-dessous :

$$\Delta G_{24} = -k_B T \left( \frac{V^{site}}{V^{\circ}} \right)$$

où «V^0» représente un volume d'état standard, «V^{site}» représente un volume d'une poche de liaison de la protéine, «T» est la température, et «k_B» est la constante de Boltzmann ;

dans lequel la variation d'énergie (ΔG_2) correspond à la somme : de la variation de l'énergie de liaison (ΔG_{21}) entre le composé et la protéine sur une distance égale ou inférieure à la distance (D_{th}) à un état (λ=λm), de la variation de l'énergie de liaison (ΔG_{22}) entre le composé et la protéine à un état compris entre l'état (À=Àm) et l'état (λ=λn), de la variation de l'énergie de solvatation (ΔG_{23}) entre le solvant et le composé à l'état non lié (λ=1) et un état (λ=λn), et du terme de correction (ΔG_{24}) par rapport à l'état standard du composé.

2. Procédé selon la revendication 1, dans lequel le solvant correspond à de l'eau.

3. Procédé selon l'une quelconque des revendications 1 et 2, dans lequel la distance entre le composé et la protéine est une distance entre un centre de gravité du composé et un centre de gravité d'un espace formé en liant des centres de gravité d'une pluralité de résidus d'acides aminés constituant un site de liaison dans la protéine.

4. Procédé selon l'une quelconque des revendications 1 à 3, dans lequel l'énergie libre de liaison est calculée séparément pour une interaction de Coulomb et pour une interaction de Lennard-Jones, et l'énergie libre de liaison est calculée pour l'interaction de Coulomb, et est ensuite calculée pour l'interaction de Lennard-Jones.

5. Procédé de criblage pour un composé, comprenant les étapes ci-dessous consistant à :

calculer des énergies libres de liaison d'une protéine avec chacun d'une pluralité de composés conformément au procédé de calcul de l'énergie libre de liaison selon l'une quelconque des revendications 1 à 4 ; et

sélectionner le composé sur la base des énergies libres de liaison calculées, dans lequel le composé sélectionné est un composé présentant une valeur optimale, parmi les énergies libres de liaison calculées de la pluralité de composés, en tant qu'une molécule médicamenteuse candidate pertinente pour la protéine.

6. Programme destiné à amener un ordinateur à exécuter un calcul de l'énergie libre de liaison entre un composé et une protéine dans un solvant, en mettant en œuvre les étapes ci-dessous consistant à :

calculer une énergie de solvatation (ΔG_1) entre le solvant et le composé ; et

calculer une variation d'énergie (ΔG_2) entre un état lié (λ=0), où le composé et la protéine sont liés, et un état non lié (À=l), où le composé et la protéine ne sont pas liés ;

dans lequel l'énergie libre de liaison est la somme de l'énergie de solvatation (ΔG_1) entre le solvant et le composé et de la variation d'énergie (ΔG_2) entre l'état lié (λ=0) et l'état non lié (λ=1) ; et

dans lequel l'étape de calcul de la variation d'énergie (ΔG_2) comprend les étapes ci-dessous consistant à :

déterminer une distance (D_{th}), dans laquelle un échantillonnage de structure est mis en œuvre, sur la base d'une distance entre le composé et la protéine à l'état lié (λ=0), dans lequel la distance (D_{th}) est sélectionnée parmi des distances comprises entre 90 % et 100 % d'une valeur minimale dans une distribution de fréquences d'une distance qui correspond à un résultat obtenu en simulant la distance entre le composé et la protéine à l'état lié (λ=0) ;

calculer une variation de l'énergie de liaison ($\Delta G_{21}$) entre le composé et la protéine sur une distance égale ou inférieure à la distance ($D_{th}$) à un état (À=Àm) qui correspond à un état compris entre l'état lié (λ=0) et l'état non lié (λ=1), l'état (À=Àm) incluant un état dans lequel une distance entre le composé et la protéine est égale ou inférieure à la distance ($D_{th}$) ;

calculer une variation de l'énergie de solvatation ($\Delta G_{23}$) entre le solvant et le composé à l'état non lié (λ=1) et un état (λ=λn) qui peut être considéré comme identique à l'état non lié (λ=1), l'état (λ=λn) qui peut être considéré comme identique à l'état non lié (λ=1) étant un état compris entre l'état lié (λ=0) et l'état non lié (λ=1) dans lequel il n'existe pratiquement pas d'interaction entre le composé et la protéine ;

calculer une variation de l'énergie de liaison ($\Delta G_{22}$) entre le composé et la protéine à un état compris entre l'état (À=Àm) et l'état (λ=λn), avec une interpolation à partir de l'état (À=Àm) et de l'état (λ=λn) ; et

calculer un terme de correction ($\Delta G_{24}$) par rapport à un état standard du composé, sur la base d'un volume d'un espace calculé à partir de la distance ($D_{th}$), le volume étant un volume sphérique présentant un rayon de la distance ($D_{th}$), le terme de correction ($\Delta G_{24}$) étant calculé selon la formule ci-dessous :

$$\Delta G_{24} = -k_B T \left( \frac{V^{site}}{V^{\circ}} \right)$$

où « $V^0$ » représente un volume d'état standard, « $V^{site}$ » représente un volume d'une poche de liaison de la protéine, «T» est la température, et «$k_B$» est la constante de Boltzmann ;

dans lequel la variation d'énergie ($\Delta G_2$) correspond à la somme : de la variation de l'énergie de liaison ($\Delta G_{21}$) entre le composé et la protéine sur une distance égale ou inférieure à la distance ($D_{th}$) à un état (λ=λm), de la variation de l'énergie de liaison ($\Delta G_{22}$) entre le composé et la protéine à un état compris entre l'état (À=Àm) et l'état (λ=λn), de la variation de l'énergie de solvatation ($\Delta G_{23}$) entre le solvant et le composé à l'état non lié (λ=1) et un état (λ=λn), et du terme de correction ($\Delta G_{24}$) par rapport à l'état standard du composé.

7. Programme selon la revendication 6, dans lequel le solvant correspond à de l'eau.

8. Programme selon l'une quelconque des revendications 6 et 7, dans lequel la distance entre le composé et la protéine est une distance entre un centre de gravité du composé et un centre de gravité d'un espace formé en liant des centres de gravité d'une pluralité de résidus d'acides aminés constituant un site de liaison dans la protéine.

9. Programme selon l'une quelconque des revendications 6 à 8, dans lequel l'énergie libre de liaison est calculée séparément pour une interaction de Coulomb et pour une interaction de Lennard-Jones, et l'énergie libre de liaison est calculée pour l'interaction de Coulomb, et est ensuite calculée pour l'interaction de Lennard-Jones.

10. Dispositif pour le calcul d'une énergie libre de liaison, comprenant :

un ordinateur ; et
un support d'enregistrement lisible ordinateur, où le support d'enregistrement est configuré de manière à stocker un programme qui, s'il est exécuté par un ordinateur, amène l'ordinateur à :

calculer une énergie de solvatation ($\Delta G_1$) entre le solvant et le composé ; et
calculer une variation d'énergie ($\Delta G_2$) entre un état lié (λ=0), où le composé et la protéine sont liés, et un état non lié (À=l), où le composé et la protéine ne sont pas liés ;

dans lequel l'énergie libre de liaison est la somme de l'énergie de solvatation ($\Delta G_1$) entre le solvant et le composé et de la variation d'énergie ($\Delta G_2$) entre l'état lié (λ=0) et l'état non lié (λ=1) ; et
dans lequel l'étape de calcul de la variation d'énergie ($\Delta G_2$) comprend les étapes ci-dessous consistant à :

déterminer une distance ($D_{th}$), dans laquelle un échantillonnage de structure est mis en œuvre, sur la base d'une distance entre le composé et la protéine à l'état lié (λ=0), dans lequel la distance ($D_{th}$) est sélectionnée parmi des distances comprises entre 90 % et 100 % d'une valeur minimale dans une distribution de fréquences d'une distance qui correspond à un résultat obtenu en simulant la distance entre le composé et la protéine à l'état lié (λ=0) ;

calculer une variation de l'énergie de liaison ($\Delta G_{21}$) entre le composé et la protéine sur une distance égale ou inférieure à la distance ($D_{th}$) à un état (À=Àm) qui correspond à un état compris entre l'état lié ($\lambda$=0) et l'état non lié ($\lambda$=1), l'état (À=Àm) incluant un état dans lequel une distance entre le composé et la protéine est égale ou inférieure à la distance ($D_{th}$) ;

calculer une variation de l'énergie de solvatation ($\Delta G_{23}$) entre le solvant et le composé à l'état non lié ($\lambda$=1) et un état ($\lambda$=$\lambda$n) qui peut être considéré comme identique à l'état non lié ($\lambda$=1), l'état ($\lambda$=$\lambda$n) qui peut être considéré comme identique à l'état non lié ($\lambda$=1) étant un état compris entre l'état lié ($\lambda$=0) et l'état non lié ($\lambda$=1) dans lequel il n'existe pratiquement pas d'interaction entre le composé et la protéine ;

calculer une variation de l'énergie de liaison ($\Delta G_{22}$) entre le composé et la protéine à un état compris entre l'état (À=Àm) et l'état ($\lambda$=$\lambda$n), avec une interpolation à partir de l'état (À=Àm) et de l'état ($\lambda$=$\lambda$n) ; et

calculer un terme de correction ($\Delta G_{24}$) par rapport à un état standard du composé, sur la base d'un volume d'un espace calculé à partir de la distance ($D_{th}$), le volume étant un volume sphérique présentant un rayon de la distance ($D_{th}$), le terme de correction ($\Delta G_{24}$) étant calculé selon la formule ci-dessous :

$$\Delta G_{24} = -k_B T \left( \frac{V^{site}}{V^\circ} \right)$$

où « $V^0$ » représente un volume d'état standard, « $V^{site}$ » représente un volume d'une poche de liaison de la protéine, «T» est la température, et «$k_B$» est la constante de Boltzmann ;

dans lequel la variation d'énergie ($\Delta G_2$) correspond à la somme : de la variation de l'énergie de liaison ($\Delta G_{21}$) entre le composé et la protéine sur une distance égale ou inférieure à la distance ($D_{th}$) à un état ($\lambda$=$\lambda$m), de la variation de l'énergie de liaison ($\Delta G_{22}$) entre le composé et la protéine à un état compris entre l'état (À=Àm) et l'état ($\lambda$=$\lambda$n), de la variation de l'énergie de solvatation ($\Delta G_{23}$) entre le solvant et le composé à l'état non lié ($\lambda$=1) et un état ($\lambda$=$\lambda$n), et du terme de correction ($\Delta G_{24}$) par rapport à l'état standard du composé.

11. Dispositif selon la revendication 10, dans lequel le solvant correspond à de l'eau.

12. Dispositif selon l'une quelconque des revendications 10 et 11, dans lequel la distance entre le composé et la protéine est une distance entre un centre de gravité du composé et un centre de gravité d'un espace formé en liant des centres de gravité d'une pluralité de résidus d'acides aminés constituant un site de liaison dans la protéine.

## FIG. 1

## FIG. 2

Disappearance of interaction with surroundings

## FIG. 3

## FIG. 4

## FIG. 5

## FIG. 6

FIG. 7

FIG. 8

FIG. 9

```
                    ┌──────────────────────────┐
                    │  Determination of D_th    │
                    └──────────────────────────┘
                                │
        ┌──────────────────────────┐    ┌──────────────────────────┐
        │ Calculation of ΔG_21      │    │ Calculation of ΔG_23      │
        └──────────────────────────┘    └──────────────────────────┘
```

$$\text{Determination of } D_{th}$$

$$\text{Calculation of } \Delta G_{21} \qquad \text{Calculation of } \Delta G_{23}$$

$$\text{Calculation of } \Delta G_1 \qquad \text{Calculation of } \Delta G_{22} \qquad \text{Calculation of } \Delta G_{24}$$

$$\Delta G_1 + \Delta G_{21} + \Delta G_{22} + \Delta G_{23} + \Delta G_{24}$$

FIG. 10

10

| 11 CPU | ⟷ ⟷ | Display unit 14 |
| 12 Memory | ⟷ ⟷ | Input unit 15 |
| | ⟷ | Output unit 16 |
| 13 Storage unit (hard disk) | ⟷ | I/O interface unit 17 |

System bus

18

## FIG. 11

|  |  | 1fkj | 1fkg |
|---|---|---|---|
| Example | $\Delta \tilde{G}^{C}_{Solv}$ | -34.19 (0.11) | -13.60 (0.09) |
|  | $\Delta \tilde{G}^{LJ}_{Solv}$ | 11.67 (0.12) | 7.68 (0.16) |
|  | $\Delta \tilde{G}^{C}_{Cmplx}$ | 35.44 (0.07) | -15.68 (0.05) |
|  | $\Delta \tilde{G}^{LJ}_{Cmplx}$ | 2.30 (0.11) | -0.90 (0.11) |
|  | $\Delta \tilde{G}^{V^{\circ} \rightarrow V^{site}}_{Vol}$ | -0.48 | -0.25 |
|  | $\Delta G^{\circ}(\text{Calc})$ | -11.7 (0.2) | -10.9 (0.2) |
| Experimental value | $\Delta G^{\circ}(\text{Exptl})$ | -12.7 (0.2) | -10.9 (0.1) |
| Comparative Example | $\Delta G^{\circ}$ (Ref) | -10.1 (1.2) | -10.3 (0.4) |

kcal/mol

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2003038672 A
- EP 2509015 A2
- US 2011130968 A1
- WO 2011016743 A1

**Non-patent literature cited in the description**

- **HOLT et al.** *J. Am. Chem. Soc.,* 1993, vol. 115, 9925-9938
- **WANG et al.** *Biophysical Journal,* vol. 91, 2798-2814